# EUROPEAN PATENT SPECIFICATION

(11) **EP 2 938 982 B1**
(45) Date of publication and mention of the grant of the patent: **07.11.2018**
(21) Application number: 13867205.0
(22) Date of filing: 27.12.2013
(51) Int. Cl.: G01L 1/00, G01L 5/00, G01P 15/00, A61B 5/00, G08B 21/02, G08B 25/01, G01P 15/08

(54) **MONITORING HIT COUNT FROM IMPACT EVENTS**
ÜBERWACHUNG DER TREFFERZÄHLUNG VON AUFPRALLEREIGNISSEN
SURVEILLANCE DU COMPTE DE COUPS À PARTIR D'ÉVÉNEMENTS D'IMPACT

(30) Priority: 27.12.2012 US 201261746305 P; 15.03.2013 US 201313844508
(43) Date of publication of application: 04.11.2015
(60) Divisional application: 18196114.5
(73) Proprietor: MC10, INC., Lexington, MA 02421 (US)
(72) Inventor: FASTERT, Steven, Chelmsford, Massachusetts 01886 (US); KACYVENSKI, Isaiah, Massachusetts 02493 (US); DOWLING, Kevin J., Massachusetts 01886 (US)
(74) Representative: Graham, Emma Louise
(86) International application number: PCT/US2013/077976
(87) International publication number: WO 2014/106041

(56) References cited:
- US-A1- 2002 060 633
- US-A1- 2008 253 766
- US-A1- 2011 181 418
- US-A1- 2011 184 320
- US-A1- 2012 075 096
- US-A1- 2012 077 441
- US-A1- 2012 150 453
- US-A1- 2012 150 453
- US-B1- 8 172 761

## Description

### CROSS-REFERENCE TO RELATED PATENT APPLICATIONS

This application claims priority to U.S. provisional application serial no. 61/746,305, filed December 27, 2012, entitled "Monitoring Hit Count From Impact Events," and U.S. patent application serial no. 13/844,508, filed March 15, 2013, entitled "Monitoring Hit Count From Impact Events".

### BACKGROUND OF THE DISCLOSURE

Impacts to a person can potentially cause injury. The impact can cause injury through forces such as translational or rotational motion, sudden changes in motion, and sudden changes in acceleration. Additionally, the duration of time the person is exposed to force can also greatly affect the effect an impact has on a person.

US Patent Application, Publication Number US2011/0184320 A1 discloses a system for monitoring a user's body with a sensor patch affixed to the user's body. The sensor detects a condition form the user's body and transmits a signal it to a base station which may be located in a helmet.

US Patent Application, Publication Number US2012/0150453 A1 discloses a system for classifying impacts to a human head. A calculation component calculates a plurality of parameters from an acceleration detected on the head of a human and a pattern recognition classifier is used to associate the impact with an event class.

US Patent Application, Publication Number US 2012/0075096 A1 discloses a hand held device for monitoring protective headgear and displaying a simulation of impacts to it. The device includes a device interface receiving the power data of an impact to the headgear, a processing device including a simulation module that generates a simulation of the impact and a user interface for displaying the simulation.

US Patent Application, Publication Number US2008/0253766 A1 discloses a data communication device and method for communication via optical light pulses. Each device has a light course and optical receiver and a light source from one device transmits data via optical pulses which is received by an optical receiver in another device.

US Patent Number US 8172761 B1 (2012) discloses a vital signs monitoring device comprising an article, an optical sensor, an accelerometer and a processor.

US Patent Application, Publication Number US 2011/0181418 A1 discloses a system and method for monitoring head accelerations of a plurality of athletes. In the system, a plurality of sensors located on different athletes transmit signals including an identifier and acceleration information wirelessly if the acceleration exceeds a threshold. A base unit then receives the information and outputs it to an output device.

### SUMMARY OF THE DISCLOSURE

In view of the foregoing, systems and methods are provided for monitoring the forces and impacts to an object. The systems and method disclosed herein can be used to monitor forces and impacts to a human subject. In some implementations, the system can be disposed into conformal electronics that can be coupled directly to an object or disposed on other objects such as clothing and protective gear. The system can include a storage module to allow for data to be reviewed and analyzed. In some implementations, the system can also include an indicator. In some implementations, the indicator can be used to display real time analysis of impacts made by the system.

According to the principles disclosed herein, a device for quantifying physical impacts to an object can include a data receiver to receive data indicative of a measure of a physical impact to an object. The data can be measured by a sensor coupled to a portion of the object. The sensor can be further configured to detect a measure of a physical impact to the object. The device can also include a hit count monitor. The hit count monitor can quantify data indicative of a first number of the physical impacts to the object that exceed a first predetermined threshold value of imparted energy based on the measure of the physical impact.

In an example, the object can be a body part. The body part can be a head, a foot, a chest, an abdomen, or a shoulder.

In an example, the device further includes a storage device coupled to the data receiver. The storage device can be configured to store data indicative of the first number of physical impacts that exceeds the predetermined threshold value of imparted energy.

In one example, the device can also include a transmission module to transmit the data indicative of the first number of physical impacts that exceed the predetermined threshold value of imparted energy. In some implementations, the transmission module can be a wireless transmission module.

In an example, the device can also include a processor to execute processor executable instructions to analyze data indicative of the measure of the physical impact to determine the first number of physical impacts that exceed the predetermined threshold value of imparted energy.

In certain examples, the device can also include a processor to execute processor executable instructions to compute an imparted energy based on an integral of a time variation of a linear acceleration and/or an angular acceleration of the physical impact. The data indicative of the time variation of a linear acceleration and/or an angular acceleration of the physical impact can be derived based on the measure of the physical impact to the object.

In an example, the sensor can also include an accelerometer and/or a gyroscope. In these examples, the measure of the physical impact can be computed based on a measurement from the accelerometer and/or the gyroscope.

In an example device, the processor can execute processor executable instructions to compute a value of imparted energy based on each measure of the physical impact. The processor can also compare the value of imparted energy based on each measure of the physical impact to the first predetermined threshold value of imparted energy. This can allow the processor to determine the first number of the physical impacts to the object that exceed the first predetermined threshold value of imparted energy.

In another example, the device can also include a processor to execute processor-executable instructions to increment a first cumulative number of hit counts for each detected physical impact to the object that exceeds the first predetermined threshold value of imparted energy.

In some examples, the processor can also execute processor executable instructions to store to a storage device or to transmit data indicative of the first cumulative number of hit counts. The sensor can be a flexible sensor, wherein the flexible sensor can be configured to conform to the portion of the object.

In an example, the device can include a substrate and a sensor disposed on the substrate. The substrate can be coupled to the portion of the object. In some examples, the substrate can be a flexible substrate, and the sensor can be a flexible sensor, configured to conform to the portion of the object.

In an example, the hit count monitor can further quantify data indicative of a second number of the physical impacts to the object that exceed a second predetermined threshold value of imparted energy greater than the first predetermined threshold value. In some examples, the device can also include a processor to execute processor-executable instructions to increment a second cumulative number of hit counts for each detected physical impact to the object that exceeds the second predetermined threshold value of imparted energy. The processor can also execute processor executable instructions to store to a storage device or to transmit data indicative of the second cumulative number of hit counts.

In an example, the hit count monitor can quantify data indicative of a third number of the physical impacts to the object that exceed a third predetermined threshold value of imparted energy greater than the second predetermined threshold value. The process can also execute processor-executable instructions to increment a third cumulative number of hit counts for each detected physical impact to the object that exceeds the third predetermined threshold value of imparted energy. The processor can also execute processor executable instructions to store to a storage device or to transmit data indicative of at least one of the second cumulative number of hit counts and the third cumulative number of hit counts.

In an example, the device can include a data fit module to perform a curve fit based on the data indicative of the measure of the physical impact to object. The curve fit can provide the data indicative of the first number of the physical impacts to the object that exceeded the first predetermined threshold value of imparted energy. The curve fit can be based on a head injury criterion function.

In an examples, the received data indicative of the measure of the physical impact can indicate that a measurement threshold of the sensor can be exceeded, resulting in non-measured impact data. The data fit module can perform the curve fit based on a pre-determined waveform to generate the non-measured data.

According to the principles disclosed herein, a device for quantifying physical impacts to an object can include a flexible sensor disposed on a substrate. The flexible sensor can be configured to conform to a portion of an object. The flexible sensor can be further configured to detect a measure of a physical impact to the object. The device can also include a hit count monitor. The hit count monitor can quantify data indicative of a first number of the physical impacts to the object that exceed a first predetermined threshold value of imparted energy based on the measure of the physical impact. The device can also include an indicator to display an indication of the first number of the physical impacts to the object that exceed the first predetermined threshold value of imparted energy,

In an example, the indicator can include at least one of a light-emitting device, a liquid crystal display, and an electrophoretic display. The indicator can be a light-emitting device (LED). The LED can provide the indication of the first number of the physical impacts using a modulation of a light amplitude of the LED according to a signaling code.

In an example, the modulation of the light amplitude of the LED according to the signaling code can be detectable by a human eye. In another example, the modulation of the light amplitude of the LED according to the signaling code can be detectable by an image sensor. The image sensor can be a component of a smartphone, a tablet, a slate, or an electronic reader.

In an example, the device can include a storage device coupled to the flexible sensor to store data indicative of the first number of physical impacts that exceeds the predetermined threshold value of imparted energy. The device can also include a processor to execute processor-executable instructions to increment a first cumulative number of hit counts for each detected physical impact to the object that exceeds the first predetermined threshold value of imparted energy.

In some examples, the indicator can display an indication of the first cumulative number of hit counts. The hit count monitor can also quantify data indicative of a second number of the physical impacts to the object that exceed a second predetermined threshold value of imparted energy greater than the first predetermined threshold value.

In an example, the device can also include a processor to execute processor-executable instructions to increment a second cumulative number of hit counts for each detected physical impact to the object that exceeds the second predetermined threshold value of imparted energy. In one example, the indicator can display an indication of the second cumulative number of hit counts or an indication of the second number of the physical impacts to the object that exceed a second predetermined threshold value of imparted energy.

In an example, the hit count monitor can quantify data indicative of a third number of the physical impacts to the object that exceed a third predetermined threshold value of imparted energy greater than the second predetermined threshold value.

In another example, the indicator can display an indication of the third cumulative number of hit counts or an indication of the third number of the physical impacts to the object that exceed a second predetermined threshold value of imparted energy.

According to the principles disclosed herein, a method for quantifying physical impacts to an object includes receiving data indicative of at least one physical impact to an object and quantifying data indicative of a first number of the at least one physical impacts to the object that exceed a first predetermined threshold value of imparted energy. The method also includes displaying and/or storing an indication of the first number of the physical impacts to the object that exceed the first predetermined threshold value of imparted energy

In an example, the method also includes displaying or transmitting an indication of the data indicative of the first number of physical impacts that exceed the predetermined threshold value of imparted energy.

In an example, the method can also include incrementing a first cumulative number of hit counts for each detected physical impact to the object that exceeds the first predetermined threshold value of imparted energy. The method can also include displaying or transmitting an indication of the first cumulative number of hit counts.

In an example, the method can include quantifying data indicative of a second number of the physical impacts to the object that exceed a second predetermined threshold value of imparted energy greater than the first predetermined threshold value. The method can also include incrementing a second cumulative number of hit counts for each detected physical impact to the object that exceeds the second predetermined threshold value of imparted energy.

In an example, the method further includes displaying or transmitting an indication of the second cumulative number of hit counts. The method can also include quantifying data indicative of a third number of the physical impacts to the object that exceed a third predetermined threshold value of imparted energy greater than the second predetermined threshold value.

In another example, the method includes incrementing a third cumulative number of hit counts for each detected physical impact to the object that exceeds the third predetermined threshold value of imparted energy, and displaying or transmitting an indication of the third cumulative number of hit counts.

### BRIEF DESCRIPTION OF THE DRAWINGS

The skilled artisan will understand that the figures, described herein, are for illustration purposes only. It is to be understood that in some instances various aspects of the described implementations may be shown exaggerated or enlarged to facilitate an understanding of the described implementations. In the drawings, like reference characters generally refer to like features, functionally similar and/or structurally similar elements throughout the various drawings. The drawings are not necessarily to scale, emphasis instead being placed upon illustrating the principles of the teachings. The drawings are not intended to limit the scope of the present teachings in any way. The system and method may be better understood from the following illustrative description with reference to the following drawings in which:
FIGs 1A-1D show block diagrams of example devices for measuring an impact acting on an object, according to the principles herein.
FIGs 2A-2C show block diagrams of example devices for measuring an impact acting on an object and displaying data indicative of the impact, according to the principles herein.
FIG 3 shows a flow chart of an example method for measuring an impact acting on an object, according to the principles herein.
FIG 4 shows a general architecture for a computer system that may be employed to implement various elements of the systems and methods described and illustrated herein.

### DETAILED DESCRIPTION

It should be appreciated that all combinations of the concepts discussed in greater detail below (provided such concepts are not mutually inconsistent) are contemplated as being part of the inventive subject matter disclosed herein. It also should be appreciated that terminology explicitly employed herein that also may appear in any disclosure incorporated by reference should be accorded a meaning most consistent with the particular concepts disclosed herein.

Following below are more detailed descriptions of various concepts related to, and embodiments of, inventive methods, apparatus and systems for monitoring the number of impacts from impact events. Herein, the number of physical impacts is also referred to as the "hit count." It should be appreciated that various concepts introduced above and discussed in greater detail below may be implemented in any of numerous ways, as the disclosed concepts are not limited to any particular manner of implementation. Examples of specific implementations and applications are provided primarily for illustrative purposes.

As used herein, the term "includes" means includes but is not limited to, the term "including" means including but not limited to. The term "based on" means based at least in part on.

The disclosure relates to systems, methods and apparatus that are used for quantifying physical impact events to which an object is subjected. The data indicative of physical impacts may be quantified as hit counts based on measurements by a data receiver coupled to the object impacted. The data receiver communicates with a hit count monitor, which can perform further actions on the data. For example, as described herein, the hit count monitor may record a cumulative count of impacts that have above a predetermined imparted energy value, store data, and/or transmit data. For any of the methods and device disclosed herein, the object on which the physical impact occurs can be a human subject and/or a body part of the human subject. For example, in some implementations the object can be a subject's head, foot, chest, abdomen, and/or shoulder. In an example, the data receive may be configured to receive data transmitted by the sensor to provide the sensor measurement data. In example, the data receiver can be a component of a device that is integral with the sensor.

An example system and method according to the principles herein provide a device for quantifying physical impact to an object. The device can include a data receiver disposed on a substrate. The data receiver can be configured to conform to a portion of an object, and can be configured to detect a measure of a physical impact to the object. The device can also include a hit count monitor. The hit count monitor can quantify data indicative of a first number of the physical impacts to the object that exceed a first predetermined threshold value of imparted energy based on the measure of the physical impact.

According the principles disclosed herein, both the data receiver and the hit count monitor can be contained within the same device, such as, but not limited to, stand alone physical impact quantification device, a device incorporated into clothing, or a device incorporated into protective equipment. In another example, the data receiver may be integrated with a wearable and conformal device that measures the impact events. In this example, the wearable and conformal device that measures the impact events may communicate with the hit count monitor wirelessly, using LEDs, or any other communication means. In some examples, the hit count monitor can be disposed proximate to the data receiver or the hit count monitor can be a monitoring device to which the impact data collected by the data receiver is transferred.

In a non-limiting example, the systems, methods and apparatus described herein for providing a measure of hit count may be integrated with a wearable and conformal device that measures the impact events. In this example, the wearable and conformal device that measures the impact events may communicate with the hit count monitoring apparatus wirelessly or using an indicator. Non-limiting examples of indicators include LEDs or any other communication means.

In one example, the data receiver includes one or more flexible electronics for sensing indications of impacts. The electronics of the data receiver can be disposed on a flexible substrate and coupled to one another by flexible interconnects. In some examples, the data receiver is encapsulated in a flexible polymer. According to the principles herein, the flexible substrate and/or polymer can include one more of a variety of polymers or polymeric composites, including polyimides, polyesters, a silicone or siloxane (e.g., polydimethylsiloxane (PDMS)), a photo-pattemable silicone, a SU8 or other epoxy-based polymer, a polydioxanone (PDS), a polystyrene, a parylene, a parylene-N, an ultrahigh molecular weight polyethylene, a polyether ketone, a polyurethane, a polyactic acid, a polyglycolic acid, a polytetrafluoroethylene, a polyamic acid, a polymethyl acrylate, or any other flexible materials, including compressible aerogel-like materials, and amorphous semiconductor or dielectric materials. In some examples described herein, flexible electronics can refer to non-flexible electronics disposed on or between flexible substrate layers.

In the various examples described herein, the data receiver of the device can comprise at least one sensor, such as an accelerometer and/or a gyroscope. The sensor can allow the data receiver to detect impacts through a change in motion of the object to which it is coupled. In one example, the data receiver can be configured to detect acceleration, change in orientation, vibration, *g*-forces and/or falling. The data receiver can be configured to make these detections along one or more orthogonal axes. In some examples, the accelerometer and/or gyroscope can be commercially available, including "commercial off-the-shelf" or "COTS." The accelerometers may include piezoelectric or capacitive components to convert mechanical motion into an electrical signal. A piezoelectric accelerometer may exploit properties of piezoceramic materials or single crystals for converting mechanical motion into an electrical signal. Capacitive accelerometers can employ a silicon micro-machined sensing element, such as a micro-electrical-mechanical system, or MEMS, sensing element. A gyroscope can facilitate the determination of refined location and magnitude detection. As a non-limiting example, a gyroscope can be used for determining the tilt or inclination of the object to which it is coupled. As another example, the gyroscope can be used to provide a measure of the rotational velocity or rotational acceleration of the object. For example, the tilt or inclination can be computed based on integrating the output (*i.e*., measurement) of the gyroscope.

In some examples, the impacts the data receiver can detect include, but are not limited to, physical impacts that can be experienced during athletic activities, such as but not limited to contact sports, noncontact sports, team sports and individual sports. For example, the physical impacts can include impacts caused by tackles in American football and the impact a baseball batter can receive when hit by a pitch. This can occur during games, athletic events, training and related activities. Other examples of impacts can include impacts caused during construction work (or other industrial work), military activity, occupation therapy, and/or physical therapy.

In various examples described herein, an impact can be quantified by the data received from the data receiver, such as, but not limited to peak acceleration data and/or force data. In another examples, an impact can be quantified based on the impact's imparted energy. In some implementations, the imparted energy is based on the integral of a time variation of a liner and/or acceleration responsive to an impact. Accordingly, the imparted energy calculation can take into account the impact's magnitude and duration.

In any of the examples disclosed herein, the device can save, transmit, and/or display data or imparted energy values. A hit count can be used to provide an indication of the number of times an object has been subjected to an impact event. In a non-limiting example, the hit count can be considered similar to pitch count used in Little League Baseball. In a non-limiting example, the hit count may be quantified as a measure of the number of impact events above a certain threshold value, or threshold values. In an example, a device may count the hits and/or provide data measurements of the impact events.

Referring again to the above described thresholds, a first predetermined threshold can be set to not register impacts deemed safe and/or below a specific threshold. In some implementations, the device's sensors may be sensitive enough to detect the impact of walking or other non-dangerous impacts, such as a safe tackle in a football game. In this example, the limit of the first threshold can be set such that it does not include these "artifact hits" in cumulative count of impacts. In one example, the device can have a plurality of thresholds such that the severity of impacts may be counted and categorized. For example, an impact above the first threshold but below a second threshold can be categorized as a mild impact, an impact above the second threshold but below a third impact can be categorized as a medium impact, and impact above the third threshold can be categorized as a severe impact. In another example, the measured of impacts events based on the systems and methods described herein could be used by a medical or other similarly qualified practitioner as a factor in an overall analysis for determining whether an impact can be categorized as non-concussive, likely-concussive, and concussive; graded on a numerical scale; graded as non-dangerous, mildly-dangerous, and dangerous; or be graded on similar scale.

In any example herein, the impact hit count may be quantified based on an amount of force or imparted energy of an impact. Additionally, the hit count may be quantified based on a measure of a physiological data representative of a physiological condition of the subject, such as but not limited to a blood pressure, a heart rate, an electrical measurement of the subject's tissue, or a measurement of a device proximate to the subject's body (including an accelerometer, a gyro, a pressure sensor, or other contact sensor).

According to the principles disclosed herein, the device for quantifying physical impacts on an object can include a storage device. The storage device can be configured to store hit counts and/or data indicative of impacts. The storage device can comprise flash memory, solid state drives, removable memory cards, or any combination thereof.

In another example, the device for quantifying physical impacts on an object can include a transmission module. The transmission module can be configured to transmit the data indicative of an impact and/or hit count to an external device. For example, the transmission module can transmit the data to a computer program running on a tablet, smartphone, or computer. In another example, the transmission module can transmit data and/or hit counts to an external hit count monitor device.

In one example, the device also includes a processor that executes processor-executable instructions. The executable instructions can include instructions to analyze physical impact data and/or to calculate imparted energy. In some examples, processor-executable instructions that cause the processor to maintain a cumulative total of the number of detected impacts. In some implementations, the cumulative total is subdivided responsive to the above described first, second, and third thresholds. According to the principles described herein, the cumulative totals can be over specific periods of time such a construction worker's shift, a specific duration of time, a game, a season, and/or a career. In some examples, the processor-executable instruction cause the processor to calculate a head injury criterion (HIC). The HIC and imparted energy can be used as a measure of the likelihood that an impact can cause a head injury.

In another example, the processor-executable instructions cause the processor to calculate the probability that an impact will have a predetermined physiological outcome. For example, after detecting and analyzing an impact, the processor may indicate to a user that the most recently detected impact has a 78% chance of causing a concussion by relating impact energy to physiological outcomes. In another example, the probability of an impact causing a predetermined physiological outcome may be categorized as a small, a medium or a high possibility of the predetermined physiological outcome occurring.

In some example implementations, the processor-executable instructions can cause the processor to perform a linear interpolation of the received data to generate data for the data points that are not measured by the data receiver. For example, the processor-executable instructions can cause the processor to perform a curve fit based on a pre-determined waveform to generate the non-measured data. In one example, the waveform can be determined based on a priori knowledge of candidate waveforms or a curve fit based on a set of known standards of the performance of low-g accelerometers for different applied forces. For example, low-g accelerometer may have a dynamic range capable of detecting up to only about 10g forces. The device may be subjected to forces outside the device's dynamic range during the course of an activity. In some example implementations, prior knowledge of candidate waveform shapes can be used to recreate a standard waveform for analysis by the hit count monitor.

In various examples described herein, the impact quantification device can be configured to include an indicator. The indicator can be used to directly display or transmit hit count and/or data indicative of an impact. In one example, the indicator provides a human readable interface, such as a screen that displays the collected data. This sequence of displayed values can be triggered but not limited to a specific action or sequence related to obtaining the displayed values such as a reset or power off and power on sequence.

In another human readable example, the indicator may include LEDs that blink or glow at a specific color to indicate the type and/or amount of impacts. In this example, the indicator can be used to blink (turn on and off) an observable sequence of light flashes that corresponds to the number of impacts above a defined threshold. A sequence of on and off flashes can be counted to give a specific number. As a non-limiting example, the sequence <on>, <off>, <on>, <off>, <on>, <off>, could correspond to 3 impacts above the threshold. For double-digits (above 9 impacts) the numbers might be indicated thusly: <on>, <off>, <pause>, <on>, <off>, <on>, <off> would correspond to 12 impacts using decimal notation. While a useful duration of the <on> pulses could be in the range of 10-400 milliseconds, any observable duration can be used. The <pause> should be perceptibly different from than the <on> signal (including being longer or shorter) to indicate the separation of numbers. This sequence of displayed values can be triggered but not limited to a specific action or sequence related to obtaining the displayed values such as a reset or power off and power on sequence.

Start and end sequences may be used to bracket the signal values such as a rapid pulsing or specific numerical values. Another numerical sequence can be used to provide a unique ID for the wearable unit.

The framework for the display of pulses can also be programmable and set up via a computer connection (wireless or wired) to tailor the sequence for specific needs. While multiple values can be communicated using longer flashing sequences, this may be less desirable due to issues of time, and complexity of interpretation. An encoding akin to a human readable Morse code-like sequence or pulse width modulation can provide more information but also may require significant training and transcription.

In yet another example, the indicator may can provide a non-human readable indicator in addition to, or in place of, the human readable indicator. For example, a smartphone application (or other similar application of machine-readable instructions on a hand-held device) can be used to read or otherwise quantify an output of an impact indicator using a camera or other means. For example, where the impact indicator provides an indication or transmits information using LEDs, the camera or other imaging component of a smartphone or other hand-held device may be used to monitor the output of the impact indicator. Examples of non-human readable interfaces using an LED include blinking the LED at a rate that cannot be perceived by the human eye, LEDs that emit electromagnetic radiation outside of the visual spectrum such as infrared or ultraviolet, and/or LEDs that glow with low luminosity such that they cannot be perceived by a human.

Non-limiting examples of hand-held devices herein include smartphones, tablets, slates, e-readers, or other portable devices, of any dimensional form factor (including mini), that can be used for collecting data (such as, but not limited to, hit count and/or measures of impact events) and/or for computing or other analysis based on the data (such as but not limited to computing the hit count, calculating imparted energy, and/or determining whether an impact event is above or below a threshold). Other devices can be used for collecting the data and/or for the computing or other analysis based on the data, including computers or other computing devices. The hand-held devices, computers and/or computing devices can be networked to facilitate greater accessibility of the collected data and/or the analyzed data, or to make it generally accessible.

As a non-limiting example, an impact indicator unit can be power cycled, and when it turns back on after a reset, it provides a series of perceptible blinks that correspond to numerical values representing the hit count. This provides a direct visual means to read the hit count. The indication, both in human readable and non-human readable configurations, may provide information relating to the total number of hits, the number of hits within specific thresholds, the number of hits over a given time duration. In another example, the indicator may also provide user profile information, such as, but not limited to, unique device ID, user name, height, weight, gender, user ID, user profile, and current threshold levels.

In another non-limiting example, the indicator is used that shows the number of hit counts such as a multi-segment display, such as but not limited to a seven segment display, LED array or LCD display. In another non-limiting example, a device such as a smart phone is used that can detect direct optical signals from the impact indicator and read the hit count.

In another non-limiting example, the hit count monitor is a reader application including a smartphone-, tablet-, or slate-based application, that reads the LED display from an indicator, calculates tiered hit counts from tiered indications of the impact indicator, and logs the data to the memory of the hit count monitor. In a non-limiting example, the tiered indication may be a green light indication for an impact that reaches a first threshold impact (including a low-level impact), a yellow light indication for an impact that reaches a second threshold impact (including a mid-level impact), and red light indication for an impact that reaches a third threshold impact (such as a high-level impact), or any combination thereof. The application can then display the counts, or indicate number of recommended hits remaining. In an example where the subject is an athlete, the hit count monitor may provide an indication of the recommended remaining hits for a player for that specific game, for the season, for the career, *etc.* In an example where the subject is a component of an instrument or device, the hit count monitor may provide an indication of the recommended remaining hits before the component is replaced. The application can also send data and reports to selected recipients such as parents, trainers, coaches, and medical professionals. The data can also be aggregated over time to provide statistics for individual players, groups of players, entire teams or for an entire league. Such data can be used to provide information indicative of trends in game play, effects of rule changes, coaching differences, differences in game strategy, and more.

In any example provided herein where the subject is an individual, it is contemplated that the system, method or apparatus has obtained the consent of the individual, where applicable, to transmit such information or other report to a recipient that is not the individual prior to performing the transmission.

Wearable electronics devices can be used to sense information regarding particular impact events (including other physiological measures). Such impact indicator devices, including units that are thin and conformal to the body, can provide this information to users and others in a variety of ways. Some non-limiting examples include wireless communication, status displays, haptic and tactile devices, and optical communication. In the case of an impact indicator, such as that described in U.S. Patent Application No. 12/972,073, 12/976,607, 12/976,814, 12/976,833, and/or 13/416,386, the wearable electronics device can be used to register and store numbers of impacts (including other physiological data) onboard.

This disclosure describes a means to display and communicate the number or level of impacts (including other physiological data) using an indicator or display. The number of impacts above a defined threshold (such as but not limited to a G-Force or HIC value) can be incremented in machine readable instructions (including software) and later, upon a specific trigger, be communicated to a user or other interested party.

As a non-limiting example of a smart lighting devices that may be applicable to a hit count monitor according to the principles described herein, U.S. Patent 6,448,967, titled "Universal Lighting Network Methods and Systems," described a device that is capable of providing illumination, and detecting stimuli with sensors and/or sending signals. The smart. lighting devices and smart lighting networks may be used for communication purposes .

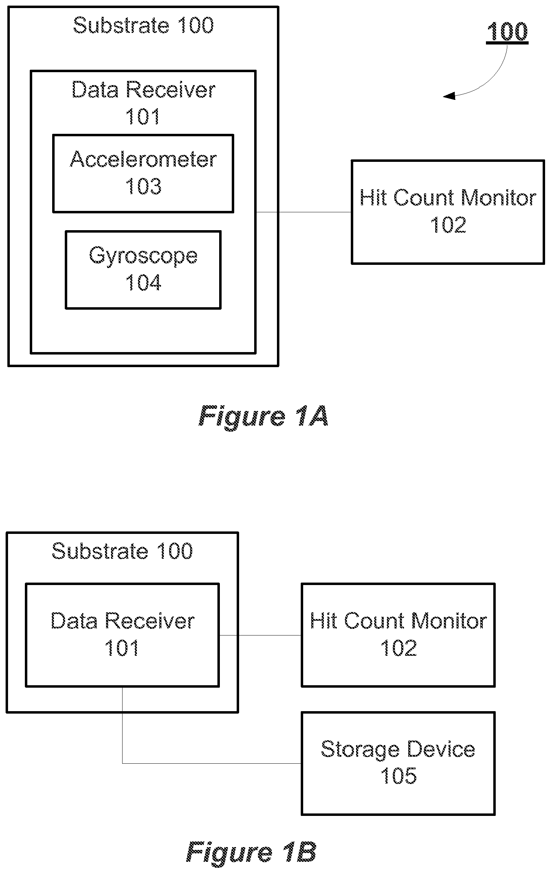

FIGs 1A-1D show non-limiting examples of possible device configurations for quantifying physical impacts to an object. The example device of FIG 1A includes a data receiver 101 disposed on a substrate 100. The data receiver 101 can be configured to conform to a portion of the object to which it and the substrate are coupled. The data receiver 101 can include one or more of any sensor component according to the principles of any of the examples and/or figures described herein, and in this example includes at least one accelerometer 103 and at least one gyroscope 104. The at least one accelerometer 103 and gyroscope can be used to measure data indicative of a physical impact to an object. The example device of FIG. 1A also includes a hit count monitor 102. The hit count monitor 102 can be configured to quantify the data that is indicative of a physical impact. In one example, the hit count monitor 102 can be disposed on the substrate 100 with the data receiver 101, and in another example, the hit count monitor 102 is disposed proximate to the substrate 100 and data receiver 101.

In the example implementation of the device in FIG. 1A, the hit count monitor 102 can be configured to quantify the data indicative of the physical impact by calculating an energy imparted and/or HIC value for the impact.

FIG. 1B shows another example device according to the principles disclosed herein that includes a substrate 100, data receiver 101, a hit count monitor 102, and a storage module 107. The storage module 107 can be configured to save data from the data receiver 101 and/or the hit count monitor 102. In some implementations the storage device 107 is any type of non-volatile memory. For example, the storage device 107 can include flash memory, solid state drives, removable memory cards, or any combination thereof. In certain examples, the storage device 107 is removable from the device. In some implementations, the storage device 107 is local to the device while in other examples it is remote. For example, the storage device 107 can be internal memory of a smartphone. In this example, the device may communicate with the phone via an application executing on the smartphone. In some implementations, the sensor data can be stored on the storage device 107 for processing at a later time. In some examples, the storage device 107 can include space to store processor-executable instructions that are executed to analyze the data from the data receiver 101. In other examples, the memory of the storage device 107 can be used to store the measured data indicative of an impact of cumulative hit counts.

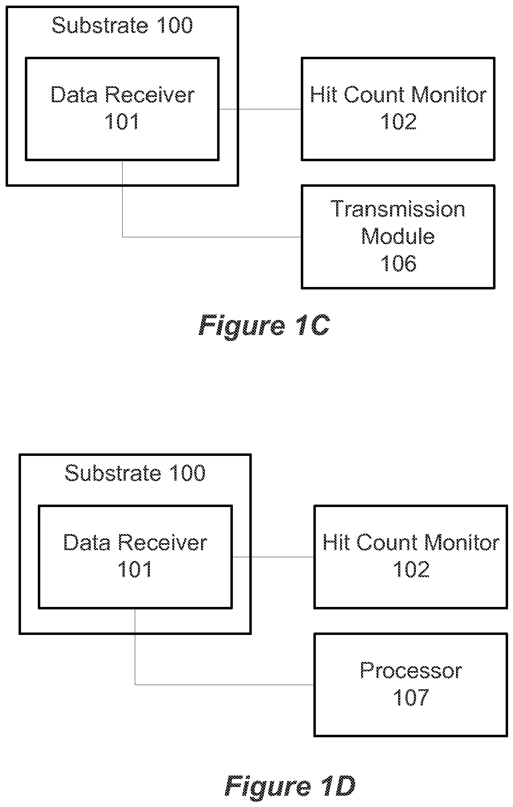

FIG. 1C shows an example device according to the principles disclosed herein that includes a substrate 100, a data receiver 101, a hit count monitor 102, and a transmission module 106. The transmission module 106 can be configured to transmit data from the data receiver 101, the hit count monitor 102, or stored in the storage device 107 to an external device. In one example, the transmission module 106 can be a wireless transmission module. For example, the transmission module 106 can transmit data to an external device via wireless networks, radio frequency communication protocols, Bluetooth, near-field communication, and/or optically using infrared or non-infrared LEDs.

FIG. ID shows an example system that includes a substrate 100, a data receiver 101, a hit count monitor 102 and a processor 107. The data receiver 101 can receive data related to sensor measurement from a sensor. In an example, the sensor can be a flexible sensor. The processor 107 can be configured to executed processor-executable instructions stored in a storage device 107 and/or within the processor 107 to analyze data indicative of a measure of a physical impact. In some implementations, the data can be directly received from the data receiver 101 or retrieved from the storage device 107. In one example, the processor can be a component of the hit count monitor 102 and/or disposed proximate to the data receiver 101. In another example, the processor 107 can be external to the device, such as in an external device that downloads and analyzes data retrieved from the device. The processor 107 can execute processor-executable instructions that quantifies the data received by the data receiver 101 in terms of imparted energy.

In another example, the processor 107 can categorize the impacts relative to predetermined thresholds. In some examples, the processor 107 can maintain hit counts for each of the bins created by the predetermined threshold and increment the counts when an impact is detected corresponding to a specific bin. For example, a first bin may include the impacts of a specific imparted energy above a first threshold but below a second threshold, a second bin may include the impacts with an imparted energy value above the second threshold but below a third threshold, and a third bin may include any impacts with an imparted energy value above the third threshold. The processor 107 may transmit the cumulative hit counts for each bin to an external device via the transmission module 106. The hit counts for each bin can be reset at predetermined intervals. For example, the device may track the number of hits an athlete receives over a time period. In another example, the cumulative hit count may indicate the number of hits remaining for a predetermined time period. For example, the device may indicate that a football player is allowed three additional hits of medium severity before the player has to be benched for the remained of the game.

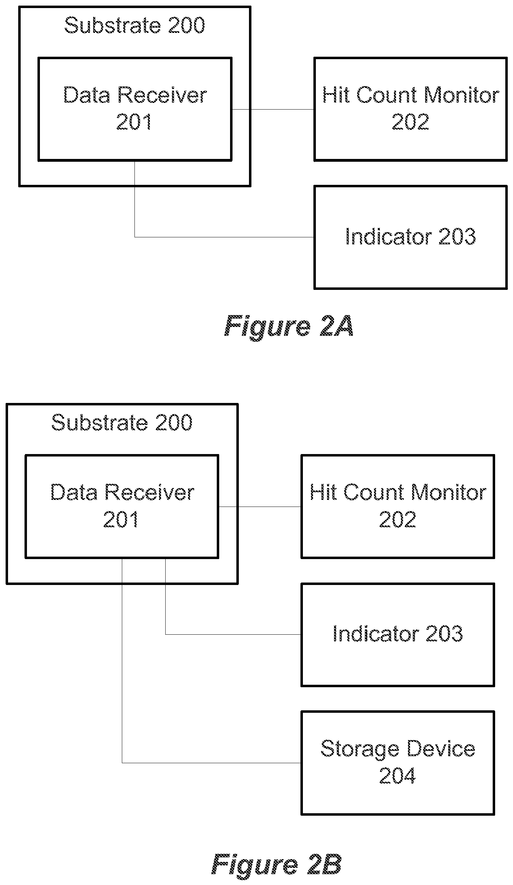

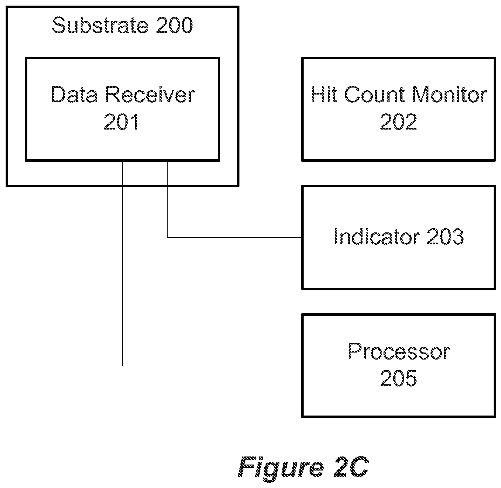

FIGs. 2A-2C show non-limiting examples of possible device configurations for quantifying physical impacts to an object and displaying the quantification of the physical impacts. The examples of FIGs. 2A---2C includes a substrate 200, a flexible sensor 201, a hit count monitor 202, and an indicator 203. In different examples the device can include a processor 205, to execute the processor-executable instructions described herein; and a storage device 204 for storing processor-executable instructions and/or data from the hit count monitor 202 and/or flexible sensor 201. The example devices of FIGs 2A-2C also include an indicator 203 for displaying and/or transmit impact information, hit count information, and/or user information.

In one example, the indicator 203 can comprise a liquid crystal display, or an electrophoeretic display (such as e-ink), and/or a plurality of indicator lights. For example, the indicator 203 can include a series of LEDs. In some implementations, the LEDs range in color, such as from green to red. In this example, if an impact is over a certain predetermined threshold is detected, the red indicator light can be activated and if the impact is under the pre-determined threshold, the green indicator light can be activated. In yet another example, the intensity of the LED indicator lights can be correlated to the severity and/or number of hits. For example, the LEDs can glow with a low intensity for a mild impact and with a high intensity for a severe impact.

In another example, the LEDs of the indicator 203 may be configured to blink at a specific rate to indicate the level of the energy imparted by an impact. For example, the indicator may blink slowly for an impact over a first threshold but below a second threshold and blink at a fast rate for an impact above the second threshold. In yet another examples, the indicator 203 may blink using a signaling code, such as Morse code, to transmit the impact data. In some implementations, as described above, the signaling of the indicator 203 is detectable to the human eye and in other implementations it is not detectable by the human eye and can only be detected by an image sensor. The indicator 203 emitting light outside the viable spectrum of the human eye (*e.g*. infrared) or too dim to be detected are examples of indication methods indictable to the human eye. In some examples, the image sensor used to detect the signals outside the viewing capabilities of a human eye can be the image sensor of a smartphone, a tablet computer, a slate computer, and/or an electronic reader.

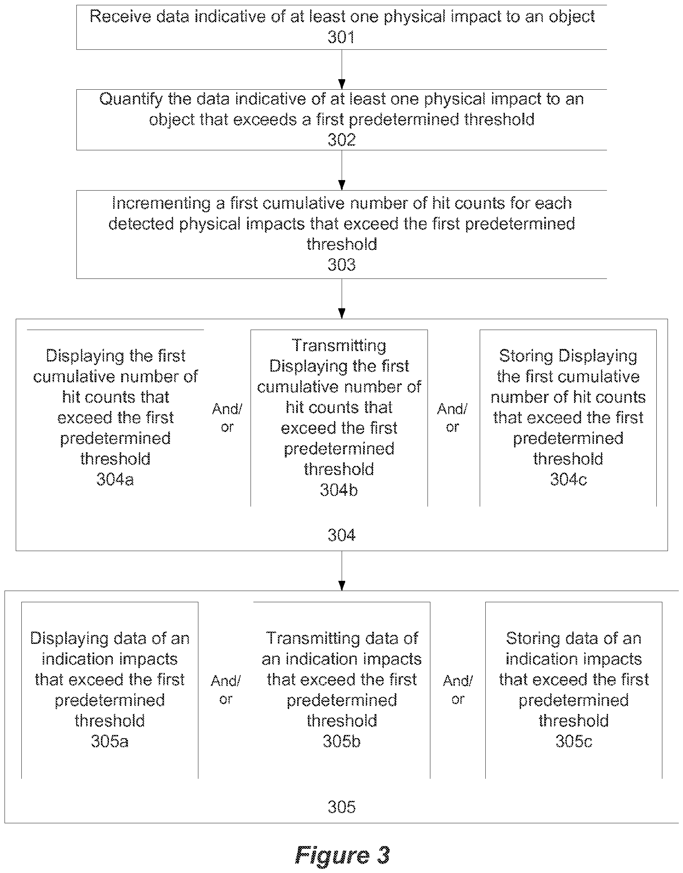

FIG. 3 show a flow chart illustrating a non-limiting example method of quantifying physical impacts on an object, according to the principles described herein.

In block 301, a processing unit receives data indicative of at least one physical impact to an object.

In block 302, the processing unit quantifies the data indicative of an impact. In one example, the processing unit may quantify the impact by a value of imparted energy. In some examples, the processing unit may only quantify impacts that have a value of imparted energy above a predetermined threshold value. As described above, in some examples, impacts that have an imparted energy value above the first predetermined threshold may be further categorized responsive to if imparted energy value of the impact exceeds a second or third predetermined threshold.

In block 303, the processing unit increments a first cumulative number of hits. In one example, the processing unit maintains a cumulative count of the number of hits that have an imparted energy value above the first predetermined threshold. In some examples, the processes maintains, and increments, second and third cumulative numbers of hits corresponding to impacts with imparted energy values above the second and third predetermined threshold, respectively.

In block 304, the device displays, transmits, or stores an indication of the first cumulative number of physical impacts. As indicated in FIG. 3, each of the steps 304a, 304b, and 304c can be performed alone or in combination. In one example, the indicator 203 can be used to display the cumulative number of hits to a user or external monitor. In another example, the transmitter 106 can be used to transmit, wirelessly or wired, to an external monitor, and in yet another example, the cumulative number of hits can be stored either locally to the device or on a separate device.

In block 305, the device displays, transmits, or stores an indication of the data indicative of the first number of physical impacts. As indicated in FIG. 3, each of the steps 305a, 305b, and 305c can be performed alone or in combination. In one example, the indicator 203 can be used to display the data indicative of an impact a user or external monitor. For example, the device may have a display that displays a graph of impact data over time to a user. In another example, the transmitter 106 can be used to transmit, wirelessly or wired, the data indicative of an impact. In such an example, the data can be downloaded from the device and analyzed by the user via a computer application. In yet another example, the data indicative of an impact can be stored either locally to the device or on a separate device, such as a laptop's hard-drive.

While the discussion herein refers to three different predetermined thresholds, it is understood that the system can assess impact events based on many more specified threshold levels according to the example methods described herein.

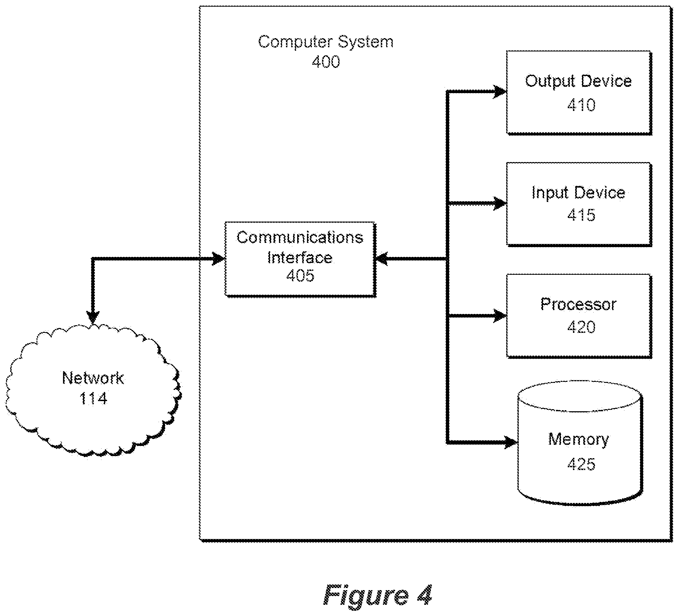

FIG. 4 shows the general architecture of an illustrative computer system 400 that may be employed to implement any of the computer systems discussed herein. The computer system 400 of FIG. 4 comprises one or more processors 420 communicatively coupled to memory 425, one or more communications interfaces 405, and one or more output devices 410 (e.g., one or more display units) and one or more input devices 415.

In the computer system 400 of FIG. 4, the memory 425 may comprise any computer-readable storage media, and may store computer instructions such as processor-executable instructions for implementing the various functionalities described herein for respective systems, as well as any data relating thereto, generated thereby, or received via the communications interface(s) or input device(s). The processors) 420 shown in FIG. 4 may be used to execute instructions stored in the memory 425 and, in so doing, also may read from or write to the memory various information processed and or generated pursuant to execution of the instructions.

The processor 420 of the computer system 400 shown in FIG. 4 also may be communicatively coupled to or control the communications interface(s) 405 to transmit or receive various information pursuant to execution of instructions. For example, the communications interface(s) 405 may be coupled to a wired or wireless network, bus, or other communication means (shown as example network 114) and may therefore allow the computer system 400 to transmit information to and/or receive information from other devices (e.g., other computer systems). While not shown explicitly in the system of FIG. 1, one or more communications interfaces facilitate information flow between the components of the system 100. In some implementations, the communications interface(s) may be configured (e.g., via various hardware components or software components) to provide a website as an access portal to at least some aspects of the computer system 400.

The output devices 410 of the computer system 400 shown in FIG. 4 may be provided, for example, to allow various information to be viewed or otherwise perceived in connection with execution of the instructions. The input device(s) 415 may be provided, for example, to allow a user to make manual adjustments, make selections, enter data or various other information, or interact in any of a variety of manners with the processor during execution of the instructions.

Embodiments of the subject matter and the operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer programs, i.e., one or more modules of computer program instructions, encoded on computer storage medium for execution by, or to control the operation of, data processing apparatus. The program instructions can be encoded on an artificially generated propagated signal, e.g., a machine-generated electrical, optical, or electromagnetic signal, that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

The operations described in this specification can be implemented as operations performed by a data processing apparatus on data stored on one or more computer-readable storage devices or received from other sources.

The term "data processing apparatus" or "computing device" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them.

A computer program (also known as a program, software, software application, script, application or code) can be written in any form of programming language, including compiled or interpreted languages, declarative or procedural languages, and it can be deployed in any form, including as a stand alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatuses can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), for example. Devices suitable for storing computer program instructions and data include all forms of non volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube), plasma, or LCD (liquid crystal display) monitor, for displaying information to the user and a keyboard and a pointing device, e.g., a mouse, touch screen or a trackball, by which the user can provide input to the computer. Other kinds of devices can be used to provide for interaction with a user as well, for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system such as system 400 or system 100 can include clients and servers. A client and server are generally remote from each other and typically interact through a communication network. The relationship of client and server arises by virtue of computer programs running on the respective computers and having a client-server relationship to each other. In some embodiments, a server transmits data to a client device (e.g., for purposes of displaying data to and receiving user input from a user interacting with the client device). Data generated at the client device (e.g., a result of the user interaction) can be received from the client device at the server.

While this specification contains many specific implementation details, these should not be construed as limitations on the scope of any inventions or of what may be claimed, but rather as descriptions of features specific to particular embodiments of the systems and methods described herein. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In some cases, the actions recited in the claims can be performed in a different order and still achieve desirable results. In addition, the processes depicted in the accompanying figures do not necessarily require the particular order shown, or sequential order, to achieve desirable results.

In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

## Claims

1. A device for quantifying physical impacts to an object, the device comprising:
a data receiver (101) configured to receive data indicative of a measure of a physical impact to an object, wherein the data is measured by a sensor that is disposed on a substrate (100), wherein the substrate (100) is coupled to a portion of the object, and wherein the sensor is configured to detect a measure of physical impacts to the object;
a hit count monitor (102) configured to quantify data indicative of a first number of the physical impacts to the object that exceed a first predetermined threshold value of imparted energy based on the measure of the physical impact; and
a data fit module configured to perform a curve fit based on the data indicative of the measure of the physical impact to the object, wherein some of the received data indicative of the measure of the physical impact indicates that a measurement threshold of the sensor is exceeded, and wherein the data fit module is configured to perform the curve fit based on a pre-determined waveform to generate non-measured data.

2. The device of claim 1, wherein the object is a body part, and wherein the body part is a head, a foot, a chest, an abdomen, or a shoulder.

3. The device of any preceding claim, further comprising a storage device (107) coupled to the data receiver (101), wherein the storage device (107) is configured to store the data indicative of the first number of the physical impacts that exceed the first predetermined threshold value of imparted energy.

4. The device of any preceding claim, further comprising a transmission module (106) configured to transmit the data indicative of the first number of the physical impacts that exceed the first predetermined threshold value of imparted energy.

5. The device of any preceding claim, further comprising a processor (107) configured to execute processor-executable instructions to analyze the data indicative of the measure of the physical impact to determine the first number of the physical impacts that exceed the predetermined threshold value of imparted energy.

6. The device of any preceding claim, further comprising a processor (107) configured to execute processor-executable instructions to compute an imparted energy based on an integral of a time variation of a linear acceleration and/or an angular acceleration of the physical impact, and wherein the data indicative of the time variation of the linear acceleration and/or the angular acceleration of the physical impact is derived based on the measure of the physical impact to the object.

7. The device of any preceding claim, wherein the sensor comprises an accelerometer and/or a gyroscope, and wherein the measure of the physical impact is computed based on a measurement from the accelerometer and/or the gyroscope.

8. The device of any preceding claim, further comprising a processor (107) configured to execute processor-executable instructions to: compute a value of imparted energy based on each measure of the physical impact; and, to compare the value of imparted energy based on each measure of the physical impact to the first predetermined threshold value of imparted energy, thereby determining the first number of the physical impacts to the object that exceed the first predetermined threshold value of imparted energy.

9. The device of any preceding claim, further comprising a processor (107) configured to execute processor-executable instructions to increment a first cumulative number of hit counts for each detected physical impact to the object that exceeds the first predetermined threshold value of imparted energy.

10. The device of any preceding claim, wherein the sensor is a flexible sensor (201), wherein the flexible sensor (201) is configured to conform to the portion of the object.

11. The device of claim 1, wherein the substrate (100) is a flexible substrate, wherein the sensor is a flexible sensor (201), and wherein the flexible sensor (201) is configured to conform to the portion of the object.

12. The device of any preceding claim, wherein the hit count monitor (102) further quantifies data indicative of a second number of the physical impacts to the object that exceed a second predetermined threshold value of imparted energy greater than the first predetermined threshold value.

13. The device of claim 12, further comprising a processor (107) configured to execute processor-executable instructions to increment a second cumulative number of hit counts for each detected physical impact to the object that exceeds the second predetermined threshold value of imparted energy.

## Patentansprüche

1. Vorrichtung zum Quantifizieren physischer Aufprallereignisse auf einen Gegenstand, wobei die Vorrichtung Folgendes umfasst:
einen Datenempfänger (101), der konfiguriert ist, um Daten zu empfangen, die einen Messwert eines physischen Aufpralls auf einen Gegenstand angeben, wobei die Daten durch einen Sensor gemessen werden, der auf einem Substrat (100) angeordnet ist, wobei das Substrat (100) mit einem Abschnitt des Gegenstands verbunden ist und wobei der Sensor konfiguriert ist, um einen Messwert eines physischen Aufpralls auf den Gegenstand zu detektieren;
eine Trefferzahlüberwachungseinheit (102), die konfiguriert ist, um Daten zu quantifizieren, die eine erste Anzahl der physischen Aufprallereignisse auf den Gegenstand angeben, die einen ersten vorbestimmten Schwellenwert der übertragenen Energie auf Grundlage des Messwerts des physischen Aufpralls überschreiten; und
ein Datenanpassungsmodul, das konfiguriert ist, um eine Kurvenanpassung auf Grundlage der Daten durchzuführen, die den Messwert des physischen Aufpralls auf den Gegenstand angeben, wobei manche der empfangenen Daten, die den Messwert des physischen Aufpralls angeben, angeben, dass ein Messschwellenwert des Sensors überschritten wurde und wobei das Datenanpassungsmodul konfiguriert ist, um die Kurvenanpassung auf Grundlage einer vorbestimmten Wellenform durchzuführen, um nicht gemessene Daten zu erzeugen.

2. Vorrichtung nach Anspruch 1, wobei der Gegenstand ein Körperteil ist und wobei der Körperteil ein Kopf, ein Fuß, eine Brust, ein Abdomen oder eine Schulter ist.

3. Vorrichtung nach einem der vorangegangenen Ansprüche, die ferner eine Speichervorrichtung (107) umfasst, die mit dem Datenempfänger (101) verbunden ist, wobei die Speichervorrichtung (107) konfiguriert ist, um die Daten zu speichern, die die erste Anzahl an physischen Aufprallereignissen angeben, die den ersten vorbestimmten Schwellenwert an übertragener Energie überschreiten.

4. Vorrichtung nach einem der vorangegangenen Ansprüche, die ferner ein Übertragungsmodul (106) umfasst, das konfiguriert ist, um die Daten zu übertragen, die die erste Anzahl an physischen Aufprallereignissen angeben, die den ersten vorbestimmten Schwellenwert an übertragener Energie überschreiten.

5. Vorrichtung nach einem der vorangegangenen Ansprüche, die ferner einen Prozessor (107) umfasst, der konfiguriert ist, um prozessorausführbare Befehle auszuführen, um die Daten zu analysieren, die die erste Anzahl an physischen Aufprallereignissen angeben, die den ersten vorbestimmten Schwellenwert an übertragener Energie überschreiten.

6. Vorrichtung nach einem der vorangegangenen Ansprüche, die ferner einen Prozessor (107) umfasst, der konfiguriert ist, um prozessorausführbare Befehle auszuführen, um eine übertragene Energie auf Grundlage eines Integrals eines zeitlichen Verlaufs von einer linearen Beschleunigung und/oder einer Winkelbeschleunigung des physischen Aufpralls zu berechnen, wobei die Daten, die den zeitlichen Verlauf von der linearen Beschleunigung und/oder Winkelbeschleunigung des physischen Aufpralls angeben, auf Grundlage des Messwerts des physischen Aufpralls auf den Gegenstand abgeleitet werden.

7. Vorrichtung nach einem der vorangegangenen Ansprüche, die ferner einen Beschleunigungsmesser und/oder ein Gyroskop umfasst, wobei der physische Aufprall auf Grundlage einer Messung des Beschleunigungsmessers und/oder des Gyroskops berechnet wird.

8. Vorrichtung nach einem der vorangegangenen Ansprüche, die ferner einen Prozessor (107) umfasst, der konfiguriert ist, um prozessorausführbare Befehle auszuführen, um: einen Wert der übertragenen Energie auf Grundlage jedes Messwerts des physischen Aufpralls zu berechnen und den Wert der übertragenen Energie auf Grundlage jedes Messwerts des physischen Aufpralls mit dem ersten vorbestimmten Schwellenwert der übertragenen Energie zu vergleichen, wodurch die erste Anzahl an physischen Aufprallereignissen auf den Gegenstand bestimmt wird, welche den ersten vorbestimmten Schwellenwert der übertragenen Energie überschreiten.

9. Vorrichtung nach einem der vorangegangenen Ansprüche, die ferner einen Prozessor (107) umfasst, der konfiguriert ist, um prozessorausführbare Befehle auszuführen, um eine erste kumulative Anzahl von Trefferzahlen für jeden detektierten physischen Aufprall auf den Gegenstand, der den ersten vorbestimmten Schwellenwert der übertragenen Energie überschreitet, zu erhöhen.

10. Vorrichtung nach einem der vorangegangenen Ansprüche, wobei der Sensor ein flexibler Sensor (201) ist, wobei der flexible Sensor (201) konfiguriert ist, um dem Abschnitt des Gegenstands zu entsprechen.

11. Vorrichtung nach Anspruch 1, wobei das Substrat (100) ein flexibles Substrat ist, wobei der Sensor ein flexibler Sensor (201) ist und wobei der flexible Sensor (201) konfiguriert ist, um dem Abschnitt des Gegenstands zu entsprechen.

12. Vorrichtung nach einem der vorangegangenen Ansprüche, wobei die Trefferzahlüberwachungseinheit (102) ferner Daten quantifiziert, die eine zweite Anzahl an physischen Aufprallereignissen auf den Gegenstand angeben, die einen zweiten vorbestimmten Schwellenwert der übertragenen Energie überschreiten, der höher ist als der erste vorbestimmte Schwellenwert.

13. Vorrichtung nach Anspruch 12, die ferner einen Prozessor (107) umfasst, der konfiguriert ist, um prozessorausführbare Befehle auszuführen, um eine zweite kumulative Anzahl von Trefferzahlen für jeden detektierten physischen Aufprall auf den Gegenstand, der den zweiten vorbestimmten Schwellenwert der übertragenen Energie überschreitet, zu erhöhen.

## Revendications

1. Dispositif pour quantifier des impacts physiques sur un objet, le dispositif comprenant :
un récepteur de données (101) configuré pour recevoir des données indicatives d'une mesure d'un impact physique sur un objet, dans lequel les données sont mesurées par un capteur qui est disposé sur un substrat (100), dans lequel le substrat (100) est couplé à une partie de l'objet, et dans lequel le capteur est configuré pour détecter une mesure d'impacts physiques sur l'objet ;
un moniteur de décompte de coups (102) configuré pour quantifier des données indicatives d'un premier nombre des impacts physiques sur l'objet qui dépassent une première valeur de seuil prédéterminée d'énergie transmise sur la base de la mesure de l'impact physique ; et
un module d'ajustement de données configuré pour effectuer un ajustement de courbe sur les données indicatives de la mesure de l'impact physique sur l'objet, dans lequel certaines des données reçues indicatives de la mesure de l'impact physique indiquent qu'un seuil de mesure du capteur est dépassé, et dans lequel le module d'ajustement de données est configuré pour effectuer l'ajustement de courbe sur la base d'une forme d'onde prédéterminée pour générer des données non mesurées.

2. Dispositif selon la revendication 1, dans lequel l'objet est une partie du corps, et dans lequel la partie du corps est une tête, un pied, une poitrine, un abdomen ou une épaule.

3. Dispositif selon l'une quelconque des revendications précédentes, comprenant en outre un dispositif de stockage (107) couplé au récepteur de données (101), dans lequel le dispositif de stockage (107) est configuré pour stocker les données indicatives du premier nombre des impacts physiques qui dépassent la première valeur de seuil prédéterminée d'énergie transmise.

4. Dispositif selon l'une quelconque des revendications précédentes, comprenant en outre un module de transmission (106) configuré pour transmettre les données indicatives du premier nombre des impacts physiques qui dépassent la première valeur de seuil prédéterminée d'énergie transmise.

5. Dispositif selon l'une quelconque des revendications précédentes, comprenant en outre un processeur (107) configuré pour exécuter des instructions exécutables par un processeur pour analyser les données indicatives de la mesure de l'impact physique afin de déterminer le premier nombre es impacts physiques qui dépassent la valeur de seuil prédéterminée d'énergie transmise.

6. Dispositif selon l'une quelconque des revendications précédentes, comprenant en outre un processeur (107) configuré pour exécuter des instructions exécutables par un processeur pour calculer une énergie transmise sur la base d'une intégrale d'une variation temporelle d'une accélération linéaire et/ou d'une accélération angulaire de l'impact physique, et dans lequel les données indicatives de la variation temporelle de l'accélération linéaire et/ou de l'accélération angulaire de l'impact physique sont déduites sur la base de la mesure de l'impact physique sur l'objet.

7. Dispositif selon l'une quelconque des revendications précédentes, dans lequel le capteur comprend un accéléromètre et/ou un gyroscope, et dans lequel la mesure de l'impact physique est calculée sur la base d'une mesure provenant de l'accéléromètre et/ou du gyroscope.

8. Dispositif selon l'une quelconque des revendications précédentes, comprenant en outre un processeur (107) configuré pour exécuter des instructions exécutables par un processeur pour : calculer une valeur d'énergie transmise sur la base de chaque mesure de l'impact physique ; et, pour comparer la valeur d'énergie transmise sur la base de chaque mesure de l'impact physique à la première valeur de seuil prédéterminée d'énergie transmise, en déterminant ainsi le premier nombre d'impacts physiques sur l'objet qui dépassent la première valeur de seuil prédéterminée d'énergie transmise.

9. Dispositif selon l'une quelconque des revendications précédentes, comprenant en outre un processeur (107) configuré pour exécuter des instructions exécutables par un processeur pour incrémenter un premier nombre cumulé de décompte de coups sur l'objet, pour chaque objet physique détecté, qui dépasse la première valeur de seuil prédéterminée d'énergie transmise.

10. Dispositif selon l'une quelconque des revendications précédentes, dans lequel le capteur est un capteur flexible (201), dans lequel le capteur flexible (201) est configuré pour se conformer à la partie de l'objet.

11. Dispositif selon la revendication 1, dans lequel le substrat (100) est un substrat flexible, dans lequel le capteur est un capteur flexible (201), et dans lequel le capteur flexible (201) est configuré pour se conformer à la partie de l'objet.

12. Dispositif selon l'une quelconque des revendications précédentes, dans lequel le moniteur de décompte de coups (102) quantifie en outre des données indicatives d'un second nombre des impacts physiques sur l'objet qui dépassent une seconde valeur de seuil prédéterminée d'énergie transmise supérieure à la première valeur de seuil prédéterminée.

13. Dispositif selon la revendication 12, comprenant en outre un processeur (107) configuré pour exécuter des instructions exécutables par un processeur pour incrémenter un second nombre cumulé de décompte de coups pour chaque impact physique détecté sur l'objet qui dépasse la seconde valeur de seuil prédéterminée d'énergie transmise.
